(19) Europäisches Patentamt
European Patent Office
Office européen des brevets

(11) Publication number: 0 030 795
A2

# (12) EUROPEAN PATENT APPLICATION

(21) Application number: 80304092.2

(22) Date of filing: 14.11.80

(51) Int. Cl.³: C 07 D 241/52
C 07 D 213/20, A 61 K 31/495

(30) Priority: 06.12.79 GB 7942090

(43) Date of publication of application:
24.06.81 Bulletin 81/25

(84) Designated Contracting States:
AT BE CH DE FR GB IT LI LU NL SE

(71) Applicant: IMPERIAL CHEMICAL INDUSTRIES LIMITED
Imperial Chemical House Millbank
London SW1P 3JF(GB)

(72) Inventor: Leigh, Thomas
Deceased
G(GB)

(72) Inventor: McLoughlin, Bernard Joseph
7 Pexhill Drive
Macclesfield Cheshire(GB)

(74) Representative: Allerton, Roy et al,
Imperial Chemical Industries Limited Legal Department:
Patents Thames House North
Millbank London SW1P 4QG(GB)

## (54) Quinoxaline derivatives, processes therefor, pharmaceutical compositions, and benzene derivatives.

(57) Compounds of the formula:-

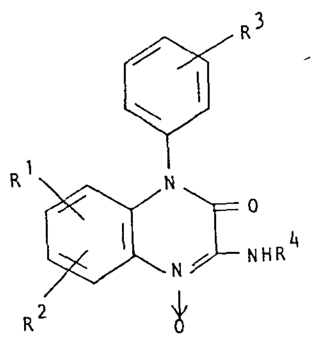

wherein $R^1$ and $R^2$, which may be the same or different, stand for hydrogen, a halogen atom or a nitro group, or a halogenoalkyl, alkyl, alkoxy or alkylthio radical of not more than 6 carbon atoms, and $R^3$ stands for hydrogen, fluorine, chlorine, bromine or methoxy, or a halogenoalkyl or alkyl radical of not more than 6 carbon atoms, provided that $R^1$, $R^2$ and $R^3$ do not all stand for hydrogen at the same time, and $R^4$ stands for hydrogen or an alkanoyl radical of not more than 6 carbon atoms. Three processes for the manufacture of said compounds. Pharmaceutical compositions comprising one of said compounds and a pharmaceutical diluent or carrier.

The compounds exhibit anticonvulsant, anti-anxiety and antipsychotic activity.

EP 0 030 795 A2

Croydon Printing Company Ltd.

QUINOXALINE DERIVATIVES, PROCESSES THEREFOR, PHARMACEUTICAL COMPOSITIONS, AND BENZENE DERIVATIVES

This invention relates to quinoxaline derivatives which possess anticonvulsant, anti-anxiety and antipsychotic properties.

The compounds of the present invention are characterised by the presence of a substituted or unsubstituted phenyl substituent on one of the nitrogen atoms in the quinoxaline nucleus, and by an oxygen atom which is linked to the other nitrogen atom in the quinoxaline nucleus. No such phenyl substituted quinoxaline derivatives are described in the prior art. However, somewhat related compounds which bear a hydrogen atom or a methyl substituent on the nitrogen atom in question are known (Journal of the Chemical Society, 1963, 2428), but they are not known to have any useful biological properties.

According to the invention there are provided quinoxaline derivatives of the formula:-

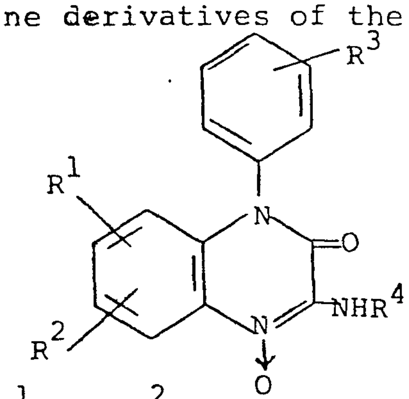

I

wherein $R^1$ and $R^2$, which may be the same or different, stand for hydrogen, a halogen atom or a nitro group or a halogenoalkyl, alkyl, alkoxy or alkylthio radical of not more than 6 carbon atoms, and $R^3$ stands for hydrogen, a fluorine, chlorine or bromine atom, a methoxy radical, or a halogenoalkyl or alkyl radical of not more than 6 carbon atoms, provided that $R^1$, $R^2$ and $R^3$ do not all stand for hydrogen at the same time, and wherein $R^4$ stands for hydrogen or an alkanoyl radical of not more

than 6 carbon atoms.

In the present specification the quinoxaline nucleus will be numbered as indicated in the following formula:-

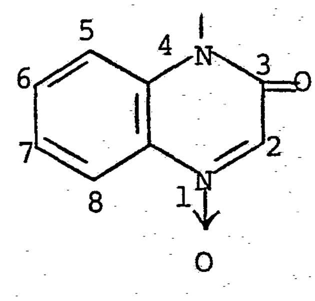

II

A suitable value for $R^1$ and/or $R^2$ is, for example, hydrogen, a fluorine, chlorine, bromine or iodine atom, a nitro group, or a trifluoromethyl, methyl, ethyl, isopropyl, n-butyl, n-hexyl, methoxy, n-butoxy, methylthio or n-propylthio radical.

A suitable value for $R^3$ is, for example, hydrogen, a fluorine, chlorine or bromine atom, a methoxy radical, or a trifluoromethyl, methyl, n-propyl or n-hexyl radical.

A suitable value for $R^4$ is, for example, hydrogen or an acetyl, propionyl, n-butyryl, isobutyryl or n-caproyl radical.

According to one embodiment of the invention there are provided compounds of the formula I wherein $R^1$ and $R^2$, which may be the same or different, stand for hydrogen, a halogen atom or a nitro group, or a halogenoalkyl, alkyl, alkoxy or alkylthio radical of not more than 6 carbon atoms, and $R^3$ stands for hydrogen, a fluorine, chlorine or bromine atom, or a halogenoalkyl radical of not more than 6 carbon atoms, provided that $R^1$, $R^2$ and $R^3$ do not all stand for hydrogen at the same time, and wherein $R^4$ stands for hydrogen or an alkanoyl radical of not more than 6 carbon atoms.

According to another embodiment of the invention there are provided compounds of the formula I wherein $R^1$ stands for a halogen atom linked to the 7-position of the quinoxaline nucleus, $R^2$ and $R^4$ stand for hydrogen, and $R^3$ stands for a fluorine, chlorine or bromine atom.

Preferred compounds of the invention are 2-amino-7-chloro-4-(4-chlorophenyl)quinoxalin-3(4H)-one-1-oxide, 2-amino-7-bromo-4-(4-chlorophenyl)quinoxalin-3(4H)-one-1-oxide, and 2-amino-7-bromo-4-(4-bromophenyl)-quinoxalin-3(4H)-one-1-oxide.

The compounds of the invention may be manufactured by any chemical process which is known to be suitable for the manufacture of chemically analogous compounds.

According to a further feature of the invention there is provided a process for the manufacture of those of the compounds of the invention wherein $R^4$ stands for hydrogen, which comprises reacting a compound of the formula:-

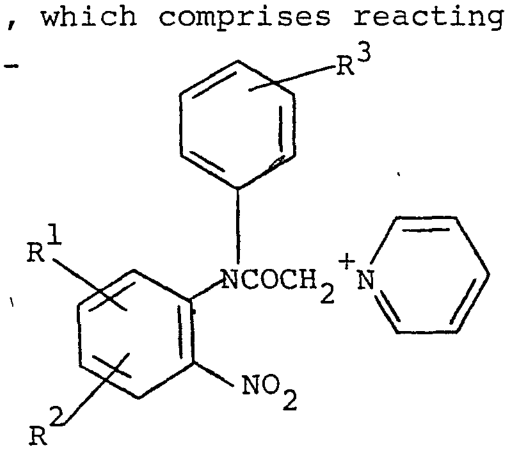

$X^-$ III

wherein $R^1$, $R^2$ and $R^3$ have the meanings stated above and $X^-$ stands for an anion, with a secondary amine in the presence of a $C_{1-5}$-alkanol, initially at a relatively low temperature.

A suitable value for $X^-$ is, for example, a halide ion, for example a chloride or bromide ion.

A suitable secondary amine is, for example, a saturated heterocyclic compound containing a 5- or 6-membered heterocyclic ring, part of which consists of the group C-NH-C, for example pyrrolidine, piperidine or _N_-methylpiperazine, or a dialkylamine of not more than 12 carbon atoms, for example dimethylamine or diethylamine. A suitable $C_{1-5}$-alkanol is, for example, methanol, ethanol or isopropanol. A suitable initial low temperature is in the range -70°C. to 0°C.

The starting materials of the formula III, which form a further feature of the invention, may be obtained by chloracetylating the corresponding _o_-nitroaniline derivative (IV; itself obtainable by conventional procedures), if necessary in the presence of a Friedel Crafts catalyst or _p_-dimethylaminopyridine, and then reacting the resulting chloracetanilide (V) with pyridine:

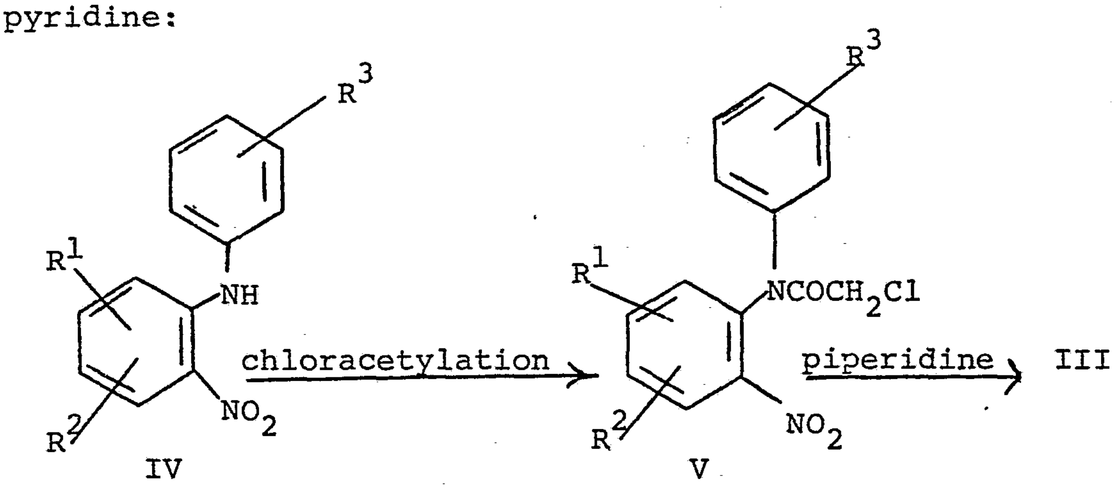


IV V III

According to a further feature of the invention there is provided a process for the manufacture of the compounds of the invention which comprises reacting a compound of the formula:-

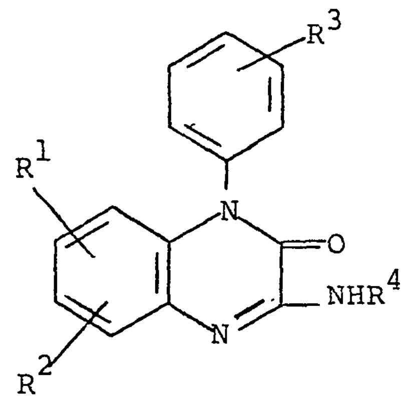

VI

wherein $R^1$, $R^2$, $R^3$ and $R^4$ have the meanings stated above, with a per-acid in the presence of an organic solvent.

A suitable per-acid is, for example, peracetic acid, and a suitable solvent is acetic acid, chloroform or methylene dichloride.

The starting materials of the formula VI, which form a further feature of the invention, may be obtained by the following sequence of reactions:-

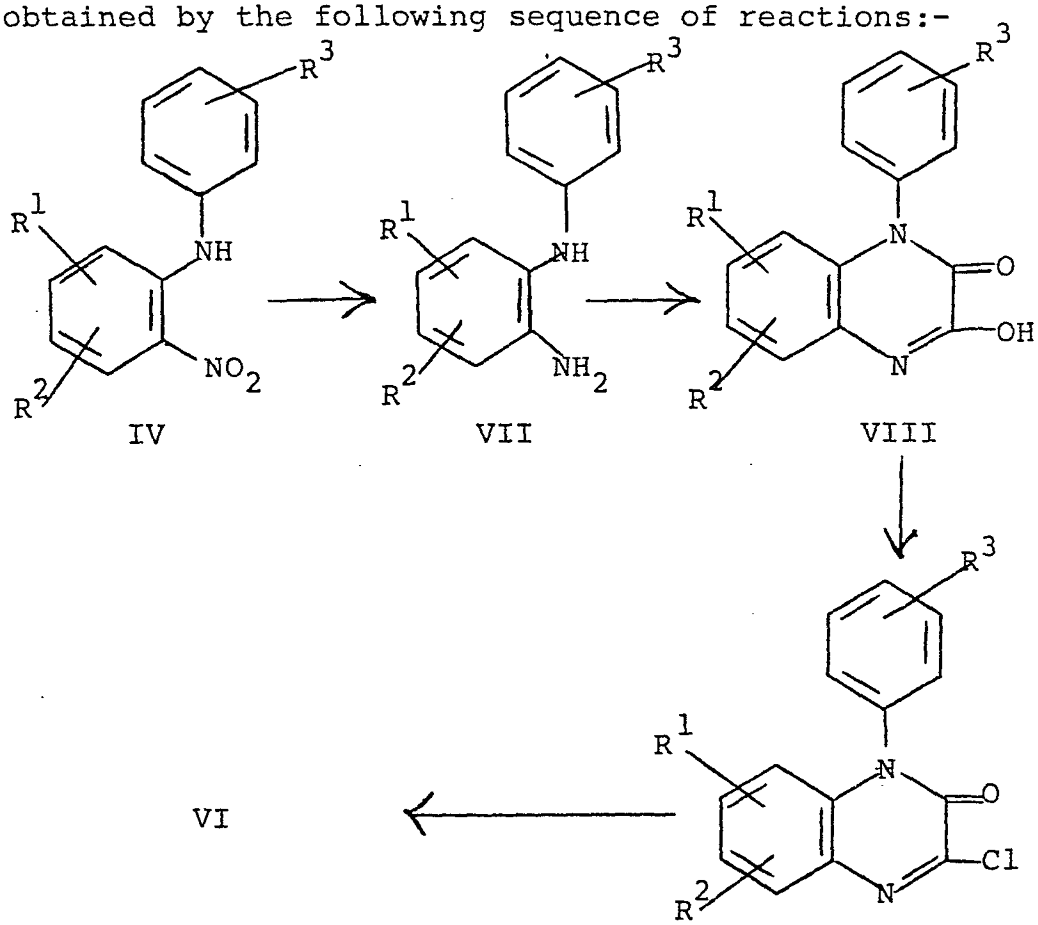

IV VII VIII IX VI

The o-nitro-aniline derivative (V) is reduced by conventional means, for example by hydrogenation in the presence of Raney nickel, and the o-phenylenediamine derivative (VII) which is thus produced is then reacted with diethyl oxalate or oxalyl chloride to give the corresponding 2-hydroxyquinoxalin—3(4H)-one derivative (VIII). The latter compound is then reacted with thionyl chloride in the presence of a very small amount of dimethylformamide to give the corresponding 2-chloro derivative (IX), and this is then reacted with the appropriate compound of the formula $R^4NH_2$ to give the desired compound of the formula VI.

According to a further feature of the invention there is provided a process for the manufacture of those of the compounds of the invention wherein $R^4$ stands for an alkanoyl radical of not more than 6 carbon atoms, which comprises reacting a compound of the formula I, wherein $R^4$ stands for hydrogen and $R^1$, $R^2$ and $R^3$ have the meanings stated above, with an acid anhydride derived from an alkanoic acid of not more than 6 carbon atoms.

As a suitable acid anhydride there may be mentioned, for example, acetic anhydride, propionic anhydride or n-butyric anhydride. The acylation may optionally be carried out in a suitable organic solvent, for example acetic acid when the acylating agent is acetic anhydride. The acylation may be accelerated or driven to completion by the application of heat.

The biological properties of the compounds of the invention have been demonstrated in the following art-recognised laboratory tests:

Anticonvulsant activity : 3-Mercaptopropionic acid induced convulsion test

This test involves seizures in mice induced by 3-mercaptopropionic acid (see, for example, Roberts and Taberner, Brit. J. Pharmacol., 1977, 61, 476P, and Adcock

and Taberner, Biochem. Pharmacol., 1978, 27, 246). In brief, the test compound is dosed orally or intraperitoneally, initially at 100mg./kg., to groups of three female mice (bodyweight 18 to 20g.). Fifty minutes after the administration of the test compound, the mice are injected intraperitoneally with 50mg./kg. of 3-mercaptopropionic acid, and the number of mice surviving 10 minutes after this injection is noted. Activity is present if the mice are protected from the lethal effect of the 3-mercaptopropionic acid.

The potency of any particular compound of the invention in this test depends upon the compound's structure, but generally speaking the compounds of the invention are active in the test at 1 to 100mg./kg.

Anti-anxiety and antipsychotic activity : Potentiation of haloperidol catalepsy

This test enables one to pick out compounds which potentiate the effects of dopamine antagonists [see, for example, Haefely et al., Adv. Biochem. Psychopharmacol., 1975, 14, 131-151 (edited by Costa and Grungard, Raven Press, New York)]. Groups of 5 female mice (bodyweight 20 to 22g.) are pre-treated with the test compound at 100mg./kg. either (a) 60 minutes prior to haloperidol administration when the test compound is dosed orally, or (b) 30 minutes prior to haloperidol administration when the test compound is dosed subcutaneously or intraperitoneally. The haloperidol is dosed intraperitoneally at a sub-cataleptic dose of 1mg./kg. (catalepsy is a condition in which there is a sudden suspension of sensation and voluntary movement, accompanied by rigidity of the whole, or certain muscles, of the body). 30 minutes later the catalepsy is assessed by placing the mice in a head-up position on a string-wrapped rod (see, for example, Doggett, Neuropharmacology, 1973, 12, 213). Mice are classed as

cataleptic if they remain stationary in a characteristic posture for at least 30 seconds, and compounds are considered active in the test if four or five of the five treated mice are cataleptic.

As indicated above, the potency of any particular compound of the invention in this test depends upon the compound's structure. Generally speaking, the compounds of the invention are active in the test at 5 to 100mg./kg.

No toxic effects have been observed with the compounds of the invention at doses at which they are active in the abovementioned tests.

When it is used to produce an anticonvulsant, anti-anxiety or antipsychotic effect in a warm-blooded animal, for example man, a compound of the invention may be administered orally at a dose of 1 to 50mg./kg., or parenterally at a dose of 1 to 50mg./kg., or rectally at a dose of 1 to 50mg./kg. In man, a total daily oral dose of 2 to 100mg.per 70 kg. is preferred. The compound of the invention may be administered once a day or several times a day.

According to a further feature of the invention there are provided pharmaceutical compositions comprising a compound of the formula I, wherein $R^1$, $R^2$, $R^3$ and $R^4$ have the meanings stated above, and an inert pharmaceutically-acceptable diluent or carrier.

The pharmaceutical compositions of the invention may be in a form suitable for oral, parenteral or rectal administration. Thus, for example, they may be in unit dosage form, for example tablets or capsules, or in injectable form, for example a sterile injectable solution or suspension, or in the form of a suppository. If desired, a composition of the invention, for example a tablet or capsule, may be a sustained release composition. The pharmaceutical compositions of the invention may be obtained in conventional manner using conventional pharmaceutically-acceptable diluents and carriers, and

they may contain, for example, up to 50% by weight of a compound of the formula I.

The compounds of the invention potentiate the effects of neuroleptic agents, for example neuroleptic phenothiazine derivatives, for example chlorpromazine, and neuroleptic butyrophenone derivatives, for example haloperidol. Accordingly, the pharmaceutical compositions of the invention may contain, in addition to a compound of the formula I, at least one known neuroleptic agent, for example a neuroleptic phenothiazine derivative or a neuroleptic butyrophenone derivative.

Without wishing to be restricted in any way by the theory, it is the Applicants' theory that the mechanism by which the compounds of the invention exert the abovementioned properties involves the fact that the said compounds are so-called "gabergic compounds". That is to say, they have the property of directly or indirectly enhancing the activity of the γ-aminobutyric acid ("GABA") in the brain.

The invention is illustrated but not limited by the following Examples:-

Example 1

N-[N-(4-chloro-2-nitrophenyl)-N-(4-chlorophenyl)-carbamoyl]methylpyridinium chloride (obtained as described below) was dissolved in methanol (100ml.). The solution was cooled to -60°C. and stirred, and piperidine (3ml.) was added dropwise. The mixture was stirred and allowed to warm up gradually to ambient temperature, and it was then stirred at ambient temperature for one hour. The resulting mixture was filtered and the solid residue was crystallised from acetic acid. There was thus obtained 2-amino-7-chloro-4-(4-chlorophenyl)quinoxalin-3(4H)-one-1-oxide, m.p. 293-6°C. (decomp.).

The pyridinium salt used as starting material

was obtained as follows:-

A solution of N-(4-chloro-2-nitrophenyl)-N-(4-chlorophenyl)-2-chloroacetamide (3g.) in dry pyridine (12ml.) was heated at 100°C. for 15 minutes. The excess of pyridine was evaporated at ca. 60°C. *in vacuo* and there was thus obtained the abovementioned pyridinium salt.

The abovementioned acetamide derivative may be obtained by either of the following methods:-

Method A

A solution of 4,4'-dichloro-2-nitrodiphenylamine (28.3g.) and 4-dimethylaminopyridine (1.2g.) in 2-chloroacetyl chloride (60ml.) was heated at 100°C. for 48 hours. The mixture was cooled, diluted with ice-cold water (500ml.), and stirred for two hours. The mixture was then shaken together with chloroform (500ml.), and the organic and aqueous layers were separated and retained. The aqueous layer was extracted with chloroform (200ml.), and the combined organic layer and chloroform extract were washed with water (2 x 200ml.) and dried ($MgSO_4$). The solvent was evaporated under reduced pressure, and the residue was crystallised from ethanol. There was thus obtained the abovementioned acetamide derivative, m.p. 143-5°C.

Method B

4,4'-Dichloro-2-nitrodiphenylamine (13.6g.) was dissolved in 2-chloroacetyl chloride (40ml.) and the solution was stirred and heated at 100°C. Freshly ground ferric chloride (1g.) was added in portions of approximately 0.1g. over 10 minutes and the mixture was then stirred and heated at 100°C. for 18 hours. The product (m.p. 143-5°C.) was isolated in the manner described in respect of Method A.

Example 2

The procedure described in Example 1 was

repeated but using an equivalent amount of N-(5-chloro-2-nitrophenyl)-N-phenyl-2-chloroacetamide (m.p. 122-4°C.; prepared using an analogous method to Method A of Example 1) in place of the acetamide derivative used in Example 1. There was thus obtained 2-amino-6-chloro-4-phenyl-quinoxalin-3(4H)-one-1-oxide, m.p. 294-6°C. (decomp.).

Example 3

The procedure described in Example 1 was repeated but using an equivalent amount of the appropriate 2-chloroacetamide to give the products shown in Table 1.

The 2-chloroacetamides used as starting materials were prepared by Method A (see Example 1); in most cases they did not crystallise but were isolated as oils (see Table 2). In these cases purification was effected as exemplified for N-(4-bromo-2-nitrophenyl)-N-(4-bromophenyl)-2-chloroacetamide:-

The crude product, obtained by Method A from 4,4'-dibromo-2-nitrodiphenylamine (1.04g.), was dissolved in ethyl acetate (10ml.) and chromatographed on Merck silica gel (Kieselgel 60, ART 7734, 150g.) made up in a 10% (by volume) solution of ethyl acetate in petroleum ether (b.p. 60-80°C.). Elution was carried out using the same solvent mixture (12 x 200ml.), and the eluates were discarded. Elution was then continued using ethyl acetate (5 x 100ml.), and these eluates were combined and the solvent was removed under reduced pressure. There was thus obtained N-(4-bromo-2-nitrophenyl)-N-(4-bromophenyl)-2-chloroacetamide as an oil which was not purified further.

TABLE 1

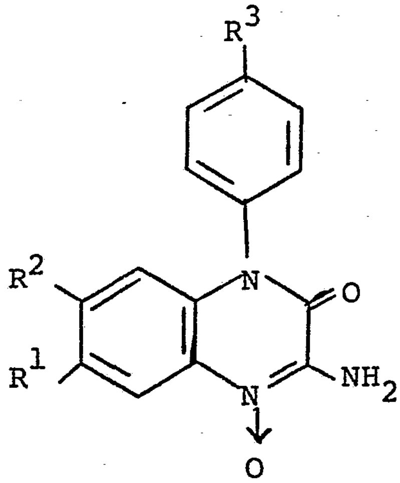

| $R^1$ | $R^2$ | $R^3$ | m.p.* | Crystallisation Solvent |
|---|---|---|---|---|
| Cl | H | Me | 282-5°C. | methanol |
| Cl | H | OMe | 299-302°C. | methanol |
| H | H | Cl | 250-252°C. | methanol |
| Cl | H | H | 288-290°C. | acetic acid |
| F | H | H | 291-294°C. | acetic acid |
| F | H | Cl | 267-270°C. | acetic acid |
| Me | H | Cl | 282-285°C. | acetic acid |
| OMe | H | Cl | 277-280°C. | acetic acid |
| F | H | F | 236-239°C. | ethyl acetate/petroleum ether (b.p. 60-80°C.) |
| Br | H | Br | 278-280°C. | acetic acid |
| $OBr^n$ | H | Cl | 218-220°C. | methanol |
| $Pr^i$ | H | Cl | 202-203°C. | ethyl acetate/petroleum ether (b.p. 60-80°C.) |
| H | Cl | Cl | 283-286°C. | ethyl acetate/petroleum ether (b.p. 60-80°C.) |
| H | OMe | H | 277.5-279.5°C. | methanol |
| H | OMe | Cl | 271-274°C. | methanol |
| H | SMe | H | 233°C. | ethyl acetate |
| H | SMe | Cl | 237.5-240°C. | methanol |

Cont..

TABLE 1 (Cont)

| $R^1$ | $R^2$ | $R^3$ | m.p.* | Crystallisation Solvent |
|---|---|---|---|---|
| H | Cl | Br | 293-294°C. | methanol |
| H | OMe | Br | 276-277°C. | methanol |
| H | Cl | I | 283-284°C. | acetic acid |
| H | OMe | I | 281-282°C. | ethyl acetate |

* All of the compounds decomposed at the melting point.

TABLE 2

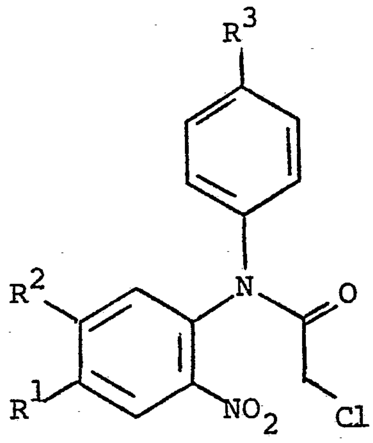

| $R^1$ | $R^2$ | $R^3$ | m.p. | Crystallisation Solvent |
|---|---|---|---|---|
| Cl | H | Me | oil | - |
| Cl | H | OMe | oil | - |
| H | H | Cl | 111-114°C. | methanol |
| Cl | H | H | 111-113°C. | methanol |
| F | H | H | 151-153°C. | ethyl acetate |
| F | H | Cl | 97-99°C. | ethanol |
| Me | H | Cl | oil | - |
| OMe | H | Cl | oil | - |
| F | H | F | 118-121°C. | ethanol |
| Br | H | Br | oil | - |
| $OBu^n$ | H | Cl | oil | - |
| $Pr^i$ | H | Cl | oil | - |
| H | Cl | Cl | 155-157°C. | methanol |
| H | OMe | H | oil | - |
| H | OMe | Cl | oil | - |
| H | SMe | H | oil | - |
| H | SMe | Cl | oil | - |
| H | Cl | Br | oil | - |
| H | OMe | Br | oil | - |

Cont ...

TABLE 2 (Cont.)

| $R^1$ | $R^2$ | $R^3$ | m.p. | Crystallisation Solvent |
|---|---|---|---|---|
| H | Cl | I | oil | - |
| H | OMe | I | oil | - |

Example 4

A suspension of 2-amino-7-bromo-4-(4-chlorophenyl)-quinoxalin-3(4H)-one (1.6g.) in chloroform (75ml.) was stirred, and 6M peracetic acid (25ml.) was added dropwise over 10 minutes. The mixture was stirred at ambient temperature for three hours, and water (100ml.) was then added. The mixture was shaken and then separated, and the organic layer was washed successively with water (50ml.) and saturated sodium chloride solution (50ml.), and then dried over anhydrous magnesium sulphate. The chloroform was removed under reduced pressure and the residue was crystallised from acetic acid. There was thus obtained 2-amino-7-bromo-4-(4-chlorophenyl)quinoxalin-3(4H)-one-1-oxide, m.p. 282-5°C. (decomp.)

The 2-amino-7-bromo-4-(4-chlorophenyl)quinoxalin-3(4H)-one used as starting material was obtained as follows:-

A solution of sodium disulphide was prepared by heating a mixture of sodium sulphide monohydrate (23.7g.), sulphur (3.2g.) and water (30ml.) at 100°C. until complete dissolution was achieved. The solution was added to a refluxing stirred suspension of 4-bromo-4'-chloro-2-nitrodiphenylamine (15.4g.) in ethanol (100ml.). Stirring and refluxing were continued for one hour. The mixture was cooled to ambient temperature and filtered and the solid residue was crystallised from ethanol. There was thus obtained 2-amino-4-bromo-4'-chlorodiphenylamine, m.p. 135°C.

A suspension of the abovementioned aminodiphenylamine derivative (7.5g.) in diethyl oxalate (45ml.) was stirred and refluxed for 18 hours. The mixture was cooled and filtered, and the solid residue was washed with cold ethanol (50ml.) and then crystallised from acetic acid. There was thus obtained 7-bromo-4-(4-chlorophenyl)-2-hydroxyquinoxalin-3-(4H)-one, m.p. above 300°C.

A mixture of the abovementioned bromoquinoxaline derivative (4.3g.), thionyl chloride (12ml.) and dimethylformamide (0.2ml.) was heated at 100°C. for 15 minutes. Excess of thionyl chloride was removed under reduced pressure and the residue, which consisted of 7-bromo-2-chloro-4-(4-chlorophenyl)quinoxalin-3(4H)-one, was dissolved in dioxan (100ml.) and added to an aqueous solution of ammonium hydroxide (100ml.; s.g. 0.910). The mixture was stirred at ambient temperature for 18 hours, filtered, and the residual solid was crystallised from acetic acid. There was thus obtained 2-amino-7-bromo-4-(4-chlorophenyl)quinoxalin-3(4H)-one, m.p. over 300°C.

Example 5

The procedure described in Example 4 was repeated but using, in place of 2-amino-7-bromo-4-(4-chlorophenyl)quinoxalin-3(4H)-one, an equivalent amount of the appropriate 2-aminoquinoxaline derivative to give the products shown in Table 3:-

TABLE 3

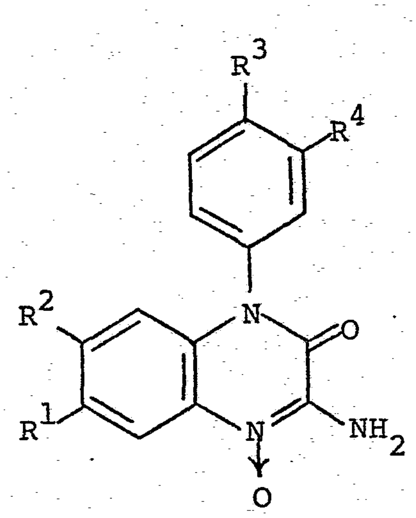

| $R^1$ | $R^2$ | $R^3$ | $R^4$ | m.p.* | Crystallisation Solvent |
|---|---|---|---|---|---|
| Cl | H | Br | H | 289-291°C. | methanol |
| Cl | H | $CF_3$ | H | 285-287°C. | ethyl acetate |
| Cl | H | F | H | 224-226°C. | ethyl acetate/petroleum ether (b.p. 60-80°C.) |
| Cl | H | H | Cl | 256-258°C. | ethyl acetate/petroleum ether (b.p. 60-80°C.) |
| $CF_3$ | H | H | H | 183-185°C. | ethanol |
| $CF_3$ | H | Cl | H | 194-195°C. | ethanol |
| $O_2N$ | H | Cl | H | 280-283°C. | ethyl acetate |
| $CF_3$ | H | $CF_3$ | H | 240-242°C. | ethyl acetate/petroleum ether (b.p. 60-80°C.) |
| Cl | Cl | Cl | H | 282-284°C. | aqueous acetic acid |
| Cl | Cl | H | H | 245-247°C. | acetic acid |

* All of the compounds decomposed at the melting point

Details on the 2-aminoquinoxaline derivatives used as starting materials are given in Table 4:

TABLE 4

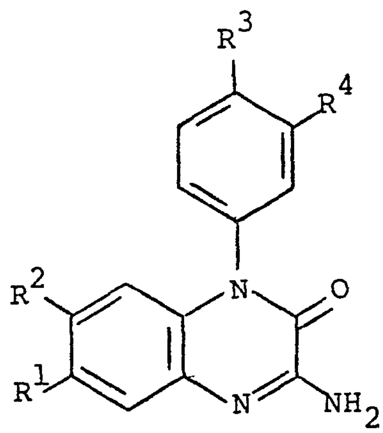

| $R^1$ | $R^2$ | $R^3$ | $R^4$ | m.p. | Crystallisation Solvent |
|---|---|---|---|---|---|
| Cl | H | Br | H | not purified further | |
| Cl | H | $CF_3$ | H | >300°C. | ethyl acetate |
| Cl | H | F | H | 258-261°C. | methanol |
| Cl | H | H | Cl | 233-235°C. | ethyl acetate/ petroleum ether (b.p. 60-80°C.) |
| $CF_3$ | H | H | H | 209°C. | ethanol |
| $CF_3$ | H | Cl | H | 245-247°C. | methanol |
| $O_2N$ | H | Cl | H | 258-260°C. | acetic acid |
| $CF_3$ | H | $CF_3$ | H | 280°C. | ethyl acetate/ petroleum ether (b.p. 60-80°C.) |
| Cl | Cl | Cl | H | 279-281°C. | ethyl acetate |
| Cl | Cl | H | H | >300°C. | ethyl acetate |

These 2-aminoquinoxaline derivatives were obtained in a similar manner to that described in Example 4 with the following exceptions:-

(a) 2-amino-4-(4-bromophenyl)-7-chloroquinoxalin-3(4H)-one was isolated as the crude product which was not further purified.

(b) 2-amino-4-(4-chlorophenyl)-6',7-dichloroquinoxalin-3(4H)-one was obtained by the following procedure:-

A mixture of 2-amino-7-chloro-4-(4-chlorophenyl)-quinoxalin-3(4H)-one-1-oxide (0.64g.), chloroacetyl chloride (3ml.) and pyridine (0.1ml.) was heated at 100°C. for 5 minutes. The mixture was cooled to ambient temperature, diluted with ether (50ml.), and stirred for 5 minutes. The mixture was filtered, the solid residue was washed with ether (2 x 25ml.), and there was thus obtained 2-(2-chloroacetylamino)-4-(4-chlorophenyl)-6,7-dichloroquinoxalin-3(4H)-one, m.p. over 300°C. This product (0.8g.) was suspended in a mixture of acetic acid (10ml.) and an aqueous solution of sulphuric acid prepared by the addition of concentrated sulphuric acid (4ml.) to water (3ml.), and the mixture was stirred and gently refluxed for 5 minutes. The mixture was cooled to ambient temperature and carefully neutralised by the addition of 2N sodium hydroxide solution. The mixture was filtered and the solid was crystallised. There was thus obtained 2-amino-4-(4-chlorophenyl)-6,7-dichloroquinoxalin-3(4H)-one.

(c) 2-amino-6,7-dichloro-4-quinoxalin-3(4H)-one was prepared as follows:-

A mixture of 2-amino-7-chloro-4-phenylquinoxalin-3(4H)-one-1-oxide (2.8g.), acetic acid (25ml.) and acetyl chloride (6ml.) was stirred and refluxed for 2 hours. The mixture was cooled to ambient temperature and diluted with water (100ml.). The mixture was

filtered, and the solid residue thus obtained was 2-acetamido-6,7-dichloro-4-phenylquinoxalin-3(4H)-one, m.p. 284-287°C. This compound (2.1g.) was hydrolysed with a mixture of concentrated sulphuric acid (8ml.), water (6ml.) and acetic acid (20ml.) in a similar manner to that described in part (b) of Example 5, to give 2-amino-6,7-dichloro-4-phenylquinoxalin-3(4H)-one.

The intermediate 2-hydroxyquinoxaline derivatives which were used are listed in Table 5:

TABLE 5

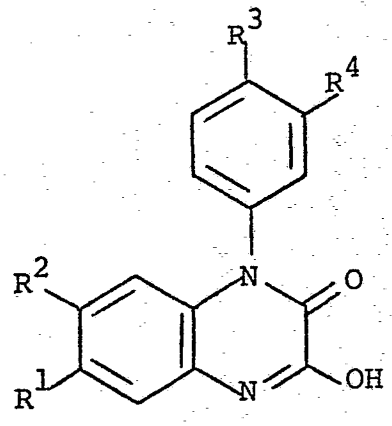

| $R^1$ | $R^2$ | $R^3$ | $R^4$ | m.p. | Crystallisation solvent |
|---|---|---|---|---|---|
| Cl | H | Br | H | >300°C. | ethyl acetate/methanol |
| Cl | H | $CF_3$ | H | >300°C. | methanol |
| Cl | H | F | H | >300°C. | - |
| Cl | H | H | Cl | >300°C. | ethyl acetate/methanol |
| $CF_3$ | H | H | H | 226-229°C. | ethanol |
| $CF_3$ | H | Cl | H | 262-264°C. | ethanol |
| $O_2N$ | H | Cl | H | >300°C. | dimethylformamide/ methanol |
| $CF_3$ | H | $CF_3$ | H | 283-285°C. | - |

They were in general prepared by the method described in Example 4 for 7-bromo-4-(4-chlorophenyl)-2-hydroxyquinoxalin-3(4H)-one, with the following exceptions:-

(a) 4-(4-chlorophenyl)-2-hydroxy-7-nitroquinoxalin-3(4H)-one was prepared as follows:-

2-Amino-4'-chloro-2-nitrodiphenylamine (7.0g.) was added in portions to stirred oxalyl chloride (45ml.). The mixture was stirred and heated for 5 minutes on the steam bath at 100°C., and then cooled and poured into an ice/water mixture (200ml.). The precipitated solid was filtered off, dried, and crystallised from acetic acid, and there was obtained 4-(4-chlorophenyl)-2-hydroxy-7-nitroquinoxalin-3(4H)-one.

(b) 2-Hydroxy-7-trifluoromethyl-4-(4-trifluoromethylphenyl)quinoxalin-3(4H)-one was obtained in the following way:-

A solution of 2-amino-4,4'-bis-trifluoromethyldiphenylamine (8.11g.) in dry toluene (200ml.) was stirred, and ethyl oxalyl chloride (3ml.) was added. The mixture was stirred at ambient temperature for 2.5 hours. Ethyl oxalyl chloride (1.0ml.) was added and stirring was continued at 50°C. for 2 hours. The mixture was cooled to 25°C., a 50% mineral oil dispersion of sodium hydride (1.4g.) was added, and the mixture was stirred and refluxed for 30 minutes. The mixture was cooled, diluted with water (200ml.) and chloroform (300ml.), and stirred for 5 minutes. It was kept for 48 hours at ambient temperature, and then filtered; both the solid residue and the filtrate being retained. The filtrate was separated, and the chloroform layer was washed with water (2 x 50ml.), dried ($MgSO_4$), and the solvent was evaporated _in vacuo_. The residue was combined with the abovementioned solid residue, the mixture was stirred together with petroleum ether (b.p. 40-60°C.), and filtered. The solid residue was washed with petroluem ether (b.p. 40-60°C.; 2 X 100ml.) and dried over silica gel in a vacuum desiccator. There was thus obtained 2-hydroxy-7-trifluoromethyl-4-(4-trifluoromethylphenyl)quinoxalin-3(4H)-one.

The appropriate 2-aminodiphenylamines are mostly known compounds, with the exception of 2-amino-4'-chloro-4-trifluoromethyldiphenylamine and 2-amino-4,4'-bis-trifluoromethyldiphenylamine, which were prepared as follows:-

(a) <u>2-amino-4'-chloro-4-trifluoromethyldiphenylamine</u>

A mixture of 4'-chloro-2-nitro-4-trifluoromethyldiphenylamine (5.3g.), Raney nickel catalyst (2.5g.) and dry ethanol (75ml.) was shaken in an atmosphere of hydrogen at ambient temperature and a pressure of one atmosphere. When no further absorption of hydrogen was observed after uptake of three equivalents of hydrogen, the hydrogen atmosphere was replaced by one of nitrogen. The mixture was filtered and the ethanol was removed from the filtrate under reduced pressure. The residue was crystallised from petroleum ether (b.p. 60-80°C.) and there was thus obtained 2-amino-4'-chloro-4-trifluoromethyldiphenylamine, m.p. 59-60°C.

(b) <u>2-amino-4,4'-bistrifluoromethyl-diphenylamine</u>

A mixture of 2-nitro-4,4'-bistrifluoromethyldiphenylamine (1.5g.), Raney nickel catalyst (1.0g.) and dry ethanol (35ml.) was treated in a smilar manner to that described immediately above, and there was thus obtained 2-amino-4,4'-bis-trifluoromethyldiphenylamine, m.p. 70-70.5°C., after crystallisation from petroleum ether (b.p. 40-60°C.).

<u>Example 6</u>

The procedure described in Example 1 was repeated except that piperidine was replaced by a molecular equivalent amount of either

(a) a 33% w/v solution of dimethylamine in ethanol, (b) pyrrolidine, (c) diethylamine, or (d) <u>N</u>-methylpiperazine. There was thus obtained 2-amino-7-chloro-4-(4-chlorophenyl)quinoxalin-3(4H)-one-1-oxide, m.p. 293-6°C. (decomp.)

Example 7

A mixture of 2-amino-7-chloro-4-(4-chlorophenyl)-quinoxalin-3(4H)-one-1-oxide (330mg.) and propionic anhydride (10ml.) was heated at 100°C. for 5 minutes. The mixture was cooled, diluted with ether (50ml.), and stirred at ambient temperature for 5 minutes. The mixture was filtered and the solid residue was dried over silica gel in a vacuum desiccator. There was thus obtained 7-chloro-4-(4-chlorophenyl)-2-propionamidoquinoxalin-3(4H)-one-1-oxide, m.p. 206-8°C.

Example 8

Example 7 was repeated, but using an equivalent amount of n-butyric anhydride in place of propionic anhydride. There was thus obtained 2-n-butyramido-7-chloro-4-(4-chlorophenyl)quinoxalin-3(4H)-one-1-oxide, m.p. 198-9°C. (decomp.).

Example 9

Example 7 was repeated, but using an equivalent amount of iscbutyric anhydride in place of propionic anhydride. There was thus obtained 7-chloro-4-(4-chlorophenyl)-2-(3-methylpropionamido)quinoxalin-3(4H)-one-1-oxide, m.p. 196-8°C. (decomp.).

Example 10

A mixture of 2-amino-7-chloro-4-(4-chlorophenyl)-quinoxalin-3(4H)-one-1-oxide (1.6g.), acetic acid (40ml.) and acetic anhydride (10ml.) was stirred and heated at 60°C. for 5 minutes. The mixture was cooled to ambient temperature and stirred for 10 minutes. Ether (100ml.) was added and stirring was continued for 5 minutes. The mixture was filtered and the solid residue was dried. There was thus obtained 2-acetamido-7-chloro-4-(4-chlorophenyl)quinoxalin-3(4H)-one-1-oxide, m.p. 207-8°C.

Example 11

Example 10 was repeated, but using an equivalent amount of 2-amino-4-(4-chlorophenyl)quinoxalin-3(4H)-one-1-oxide in place of 2-amino-7-chloro-4-(4-chlorophenyl)-quinoxalin-3(4H)-one-1-oxide. There was thus obtained

2-acetamido-4-(4-chlorophenyl)quinoxalin-3(4H)-one-1-oxide, m.p. 164-7°C. (decomp.)

Example 12

The procedure described in the first paragraph of Example 1 was repeated, except that an equivalent amount of N-[N-(4-chloro-2-nitrophenyl)-N-(4-chlorophenyl)-carbamoyl]methylpyridinium bromide was used in place of the corresponding chloride. There was thus obtained 2-amino-7-chloro-4-(4-chlorophenyl)quinoxalin-3(4H)-one-1-oxide, m.p. 293-6°C. (decomp.)

The pyridinium salt used as starting material was obtained as follows:-

A solution of 4,4'-dichloro-2-nitrodiphenylamine (5.66g.) and 4-dimethylaminopyridine (0.2g.) in 2-bromoacetyl bromide (7.5ml.) was heated at 100°C. for 18 hours. The mixture was cooled, diluted with ice-cold water (200ml.), and stirred for two hours. The mixture was then shaken together with chloroform (100ml.), the mixture was separated, and the organic phase was washed with water (3 x 50ml.), and dried ($MgSO_4$). The solvent was evaporated under reduced pressure, and the residue was crystallised from ethanol. There was thus obtained N-(4-chloro-2-nitrophenyl)-N-(4-chlorophenyl)-2-bromoacetamide, m.p. 123-5°C.

A solution of the abovementioned acetamide derivative (2.02g.) in dry pyridine (5ml.) was heated at 100°C. for 15 minutes. The excess of pyridine was evaporated at ca. 60°C. in vacuo and there was thus obtained the abovementioned pyridinium salt.

Example 13

A mixture of 2-amino-7-chloro-4-(4-chlorophenyl)-quinoxalin-3(4H)-one-1-oxide (10g.) and lactose (80g.) was sifted through a 60-mesh sieve (British Pharmacopoeia 1980, II, A195). Sufficient of a 10% w/v aqueous solution of gelatin was added to produce a stiff paste. The paste was passed through a 16-mesh sieve, dried,

and then passed through a 20-mesh sieve. Maize starch (6g.) and magnesium stearate (1g.) were thoroughly mixed with the resulting granules. The resulting mixture was compressed into tablets, each containing 10mg. of the active ingredient.

What we claim is:-

1. A quinoxaline derivative of the formula:-

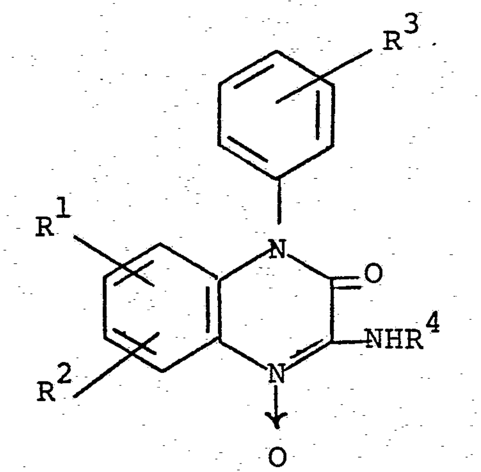

I

wherein $R^1$ and $R^2$, which may be the same or different, stand for hydrogen, a halogen atom or a nitro group, or a halogenoalkyl, alkyl, alkoxy or alkylthio radical of not more than 6 carbon atoms, and $R^3$ stands for hydrogen, a fluorine, chlorine or bromine atom, a methoxy radical, or a halogenoalkyl or alkyl radical of not more than 6 carbon atoms, provided that $R^1$, $R^2$ and $R^3$ do not all stand for hydrogen at the same time, and wherein $R^4$ stands for hydrogen or an alkanoyl radical of not more than 6 carbon atoms.

2. A quinoxaline derivative of the formula I wherein $R^1$ and $R^2$, which may be the same or different, stand for hydrogen, a halogen atom or a nitro group, or a halogenoalkyl, alkyl, alkoxy or alkylthio radical of not more than 6 carbon atoms, and $R^3$ stands for hydrogen, a fluorine, chlorine or bromine atom, or a halogenoalkyl radical of not more than 6 carbon atoms, provided that $R^1$, $R^2$ and $R^3$ do not all stand for hydrogen at the same time, and wherein $R^4$ stands for hydrogen or an alkanoyl radical of not more than 6 carbon atoms.

3. A compound as claimed in claim 2 wherein $R^1$ stands for a halogen atom linked to the 7-position of

the quinoxaline nucleus, $R^2$ and $R^4$ stand for hydrogen, and $R^3$ stands for a fluorine, chlorine or bromine atom.

4. A compound as claimed in claim 3 which is selected from the group consisting of 2-amino-7-chloro-4-(4-chlorophenyl)quinoxalin-3(4H)-one-1-oxide, 2-amino-7-bromo-4-(4-chlorophenyl)quinoxalin-3(4H)-one-1-oxide and 2-amino-7-bromo-4-(4-bromophenyl)quinoxalin-3(4H)-one-1-oxide.

5. A process for the manufacture of a compound claimed in claim 1 in which $R^4$ stands for hydrogen, characterised in that a compound of the formula:-

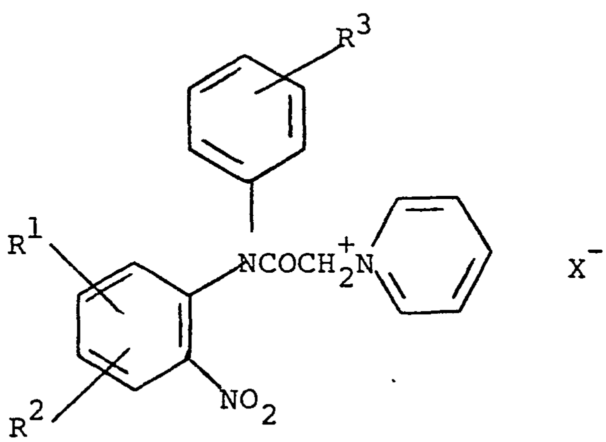

III

wherein $R^1$, $R^2$ and $R^3$ have the meanings stated in claim 1 and $X^-$ stands for an anion, is reacted with a secondary amine in the presence of a $C_{1-5}$-alkanol, initially at a relatively low temperature.

6. A process for the manufacture of a compound claimed in claim 1, characterised in that a compound of the formula:-

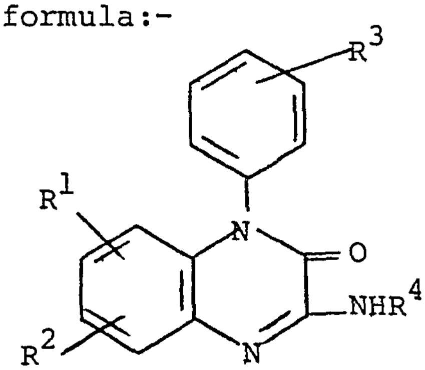

VI

wherein $R^1$, $R^2$, $R^3$ and $R^4$ have the meanings stated in claim 1, is reacted with a per-acid in the presence of an organic solvent.

7. A process for the manufacture of a compound claimed in claim 1 in which $R^4$ stands for an alkanoyl radical of not more than 6 carbon atoms, characterised in that a compound of the formula I, wherein $R^4$ stands for hydrogen and $R^1$, $R^2$ and $R^3$ have the meanings stated in claim 1, is reacted with an acid anhydride derived from an alkanoic acid of not more than 6 carbon atoms.

8. A pharmaceutical composition comprising a compound of the formula I, wherein $R^1$, $R^2$, $R^3$ and $R^4$ have the meanings stated in claim 1, and an inert pharmaceutically-acceptable diluent or carrier.

9. A pharmaceutical composition as claimed in claim 8 which contains, in addition to the compound of the formula I, at least one known neuroleptic agent.

10. A compound of the formula:-

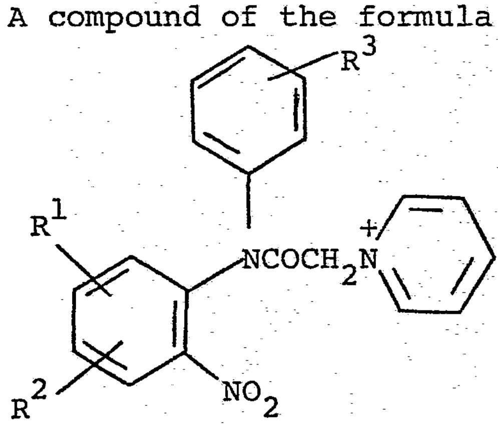

$X^-$ III

wherein $R^1$, $R^2$ and $R^3$ have the meanings stated in claim 1 and $X^-$ has the meaning stated in claim 5.

11. A compound of the formula:-

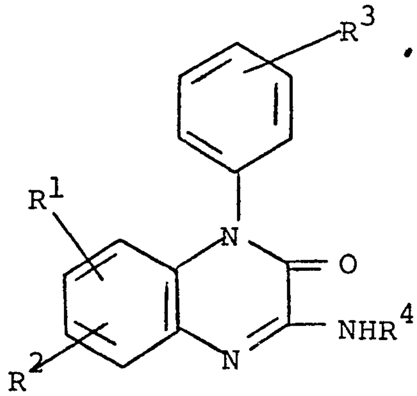

VI

wherein $R^1$, $R^2$, $R^3$ and $R^4$ have the meanings stated in claim 1.

Roy Allerton
Authorised Representative

RA/LMS : 20 Oct 80

31075/EP 0030795

CLAIMS FOR AUSTRIA.



What we claim is:-

1. A process for the manufacture of a quinoxaline derivative of the formula:-

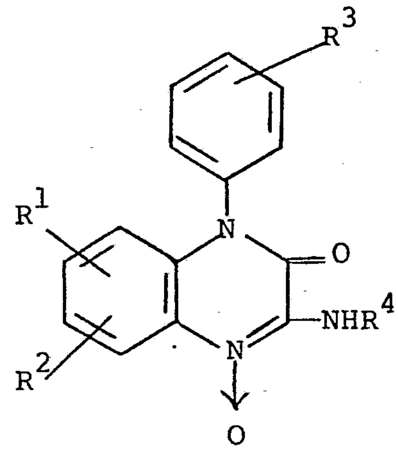

I

wherein $R^1$ and $R^2$, which may be the same or different, stand for hydrogen, a halogen atom or a nitro group, or a halogenoalkyl, alkyl, alkoxy or alkylthio radical of not more than 6 carbon atoms, and $R^3$ stands for hydrogen, a fluorine, chlorine or bromine atom, a methoxy radical, or a halogenoalkyl or alkyl radical of not more than 6 carbon atoms, provided that $R^1$, $R^2$ and $R^3$ do not all stand for hydrogen at the same time, and wherein $R^4$ stands for hydrogen, characterised in that a compound of the formula:-

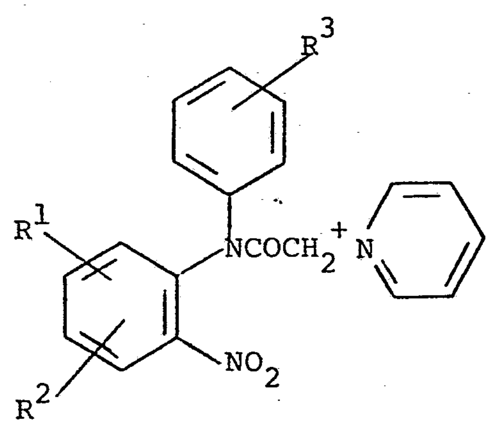

$X^-$ III

wherein $R^1$, $R^2$ and $R^3$ have the meanings stated above and $X^-$ stands for an anion, is reacted with a secondary amine in the presence of a $C_{1-5}$-alkanol, initially at a relatively low temperature.

2. A process as claimed in claim 1, characterised in that $X^-$ stands for a halide ion, the secondary amine is a saturated heterocyclic compound containing a 5- or 6-membered heterocyclic ring, part of which consists of the group C-NH-C, or a dialkylamine of not more than 12 carbon atoms, and the initial low temperature is in the range -70°C. to 0°C.

3. A process for the manufacture of a compound of the formula I, wherein $R^1$, $R^2$ and $R^3$ have the meanings stated in claim 1 and $R^4$ stands for hydrogen or an alkanoyl radical of not more than 6 carbon atoms, characterised in that a compound of the formula:-

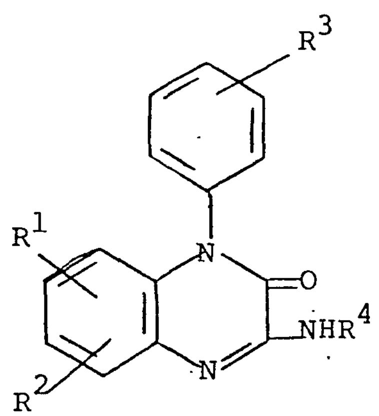

VI

wherein $R^1$, $R^2$, $R^3$ and $R^4$ have the meanings stated above, is reacted with a per-acid in the presence of an organic solvent.

4. A process as claimed in claim 3, characterised in that the per-acid is peracetic acid and the solvent is acetic acid, chloroform or methylene dichloride.

5. A process for the manufacture of a compound of the formula I, wherein $R^1$, $R^2$ and $R^3$ have the meanings

stated in claim 1 and $R^4$ stands for an alkanoyl radical of not more than 6 carbon atoms, characterised in that a compound of the formula I, wherein $R^1$, $R^2$ and $R^3$ have the meanings stated above and $R^4$ stands for hydrogen, is reacted with an acid anhydride derived from an alkanoic acid of not more than 6 carbon atoms.

6. A process as claimed in claim 5, characterised in that the acid anhydride is acetic anhydride, propionic anhydride or n-butyric anhydride.

7. A process as claimed in claim 6, characterised in that the acid anhydride is acetic anhydride and the process is carried out in the presence of acetic acid.

RA/LMS : 21 Oct 80
ICI Case PH. 31075 Europe (Austria)